# EUROPÄISCHE PATENTSCHRIFT

(11) **EP 2 224 897 B1**
(45) Veröffentlichungstag und Bekanntmachung des Hinweises auf die Patenterteilung: **17.08.2016**
(21) Anmeldenummer: 08867147.4
(22) Anmeldetag: 22.12.2008
(51) Int. Cl.: A61K 8/04, A61K 8/31, A61K 8/37, A61K 8/58, A61K 8/891, A61K 8/893, A61Q 15/00, A61K 8/894

(54) **WASSERFREIE ANTITRANSPIRANT-SPRAYS MIT VERBESSERTER WIRKSTOFFFREISETZUNG**
WATER-FREE ANTIPERSPIRANT SPRAYS WITH IMPROVED SUBSTANCE RELEASE
SPRAYS ANTI-TRANSPIRANTS SANS EAU À LIBÉRATION D'AGENTS AMÉLIORÉE

(30) Priorität: 28.12.2007 DE 102007063352
(43) Veröffentlichungstag der Anmeldung: 08.09.2010
(73) Patentinhaber: Henkel AG & Co. KGaA, 40589 Düsseldorf (DE)
(72) Erfinder: BANOWSKI, Bernhard, 40597 Düsseldorf (DE); BUSE, Nadine, 40724 Hilden (DE)
(86) Internationale Anmeldenummer: PCT/EP2008/068204
(87) Internationale Veröffentlichungsnummer: WO 2009/083547

(56) Entgegenhaltungen:
- WO-A1-96/24326
- WO-A1-03/002082
- US-A- 5 449 511
- US-A1- 2003 215 399
- US-A1- 2004 247 546
- US-B2- 6 524 562

## Beschreibung

Gegenstand der vorliegenden Erfindung sind wasserfreie schweißhemmende Zusammensetzungen, insbesondere auf Basis einer wasserfreien, treibmittelfrei oder mit einem Treibmittel versprühbaren Suspension, die eine verbesserte Wirkstofffreisetzung des schweißhemmenden Wirkstoffs ermöglichen.
Wasserfreie, mit einem Treibmittel versprühbare Antitranspirant-Suspensionen enthalten neben schweißreduzierenden Wirkstoffen in der Regel mindestens ein kosmetisches Öl als Träger für den teilchenförmigen schweißreduzierenden Wirkstoff. Die Supensionen werden in einem druckfesten Behälter, meist einer Dose aus Weißblech oder Aluminium, die innen lackiert ist, zusammen mit einem verflüssigten Kohlenwasserstoff, wie n-Butan, iso-Butan und/oder Propan, als Treibmittel abgefüllt. Vor Gebrauch des Sprühventils, bei dem Treibmittel und eine Portion der Suspension freigesetzt werden, muss der Behälter zunächst ausreichend geschüttelt werden, um den abgesetzten Antitranspirantwirkstoff aufzumischen. Damit sich der suspendierte Antitranspirantwirkstoff nicht sofort wieder absetzt, enthalten handelsübliche Suspensionen ein Suspendiermittel, z. B. hydrophob modifizierte Hectorite, wie sie beispielsweise unter der Handelsbezeichnung Bentone Gel von den Firmen Rheox und Elementis Specialties erhältlich sind.
Bei den handelsüblichen Sprays ist der in dem wasserfreien Träger suspendierte Antitranspirantwirkstoff mit einer Ölschicht bedeckt. Während und nach der Applikation auf der Haut begünstigt diese Ölschicht das Sprühbild, das heißt, der Wirkstoff wird nicht zu sehr vernebelt, sondern gelangt gezielt auf die Haut; außerdem sorgt die Ölschicht für eine gewisse Haftung des pulverförmigen Antitranspirantwirkstoffs auf der Haut Diese Ölschicht verzögert allerdings die Freisetzung des Antitranspirantwirkstoffs in die wirksame wasserlösliche Form.
US 5449511 offenbart die Verwendung der Organosiloxan-Oxyalkylen-Copolymere Dow Coming 190 und Dow Corning 193 als wasserlösliche Maskierungsmittel, um weiße Rückstände des ungelösten, suspendierten Antitranspirant-Wirkstoffs zu maskieren, ohne dabei dessen schweißhemmende Wirkung zu beeinträchtigen. Dow Corning 190 und Dow Coming 193 sind verzweigte Copolymere, bei denen EO-PO-Copolymer-Ketten auf eine Polysiloxan-Kette aufgepfropft sind.

Überraschend wurde nun gefunden, dass die Freisetzung des schweißhemmenden Wirkstoffs aus einer wasserfreien Antitranspirant-Zusammensetzung verbessert werden kann, wenn mindestens ein Organosiloxan-Oxyalkylen-Copolymer enthalten ist, das aus linearen Polysiloxan-Polyoxyalkylen-Blockcopolymeren, insbesondere aus linearen Polysiloxan-Polyoxyethylen-Polyoxy-Propylen-Blockcopolymeren, ausgewählt ist.

Gegenstand der vorliegenden Erfindung sind daher schweißhemmende Zusammensetzungen zur persönlichen Körperpflege, konfektioniert als treibmittelfrei oder mit einem Treibmittel versprühbare Suspension, enthaltend mindestens einen im Träger unlöslichen schweißhemmenden Wirkstoff, mindestens ein unter Normalbedingungen flüssiges Öl als Träger, in dem der schweißhemmende Wirkstoff a) suspendiert ist, 0 - 3 Gew.-%, bevorzugt 0 - 2 Gew.%, freies Wasser, bezogen auf das Gewicht der treibmittelfreien Zusammensetzung, und mindestens ein Organosiloxan-Oxyalkylen-Copolymer, ausgewählt aus linearen Polyrsiloxan-Polyoxyalkylen-Blockcopolymeren, insbesondere aus linearen Polysiloxan-Polyoxyethylen-Polyoxypropylen-Blockcopolymeren.
Die erfindungsgemäßen schweißhemmenden Zusammensetzungen auf wasserfreier Basis sind als treibmittelfrei, beispielsweise in einem Pumpspender, oder mit einem Treibmittel versprühbare Suspension konfektioniert.
Die erfindungsgemäße schweißhemmende Wirkstoffkombination eignet sich bevorzugt für versprühbar konfektionierte Zusammensetzungen, insbesondere für treibmittelhaltige Suspensionen, die als Antitranspirant-Spray angewendet werden.

"Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Die erfindungsgemäß geeigneten Organosiloxan-Oxyalkylen-Copolymere d) sind ausgewählt aus linearen Polysiloxan-Polyoxyalkylen-Blockcopolymeren, insbesondere aus linearen Polysiloxan-Polyoxyethylen-Polyoxypropylen-Blockcopolymeren. Erfindungsgemäß außerordentlich bevorzugt ist ein lineares Polysiloxan-Polyoxyethylen-Polyoxypropylen-Blockcopolymer mit der INCI-Bezeichnung PEG/PPG-22/24 Dimethicone. Ein derartiges lineares Polysiloxan-Polyoxyethylen-Polyoxypropylen-Blockcopolymer ist beispielsweise unter der Handelsbezeichnung Mirasil DMCO (INCI: PEG/PPG-22/24 Dimethicone) von der Firma Rhodia erhältlich.

Weitere bevorzugte erfindungsgemäße Zusammensetzungen sind dadurch gekennzeichnet, dass das Organosiloxan-Oxyalkylen-Copolymer d) einen HLB-Wert im Bereich von 8 - 20, bevorzugt 10 - 18, besonders bevorzugt 11 - 16 aufweist.

Ein Organosiloxan-Oxyalkylen-Copolymer der allgemeinen Strukturformel (II) 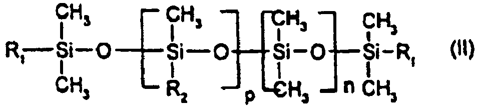 mit einem HLB-Wert im Bereich von 8 - 20, bevorzugt 10 - 18, besonders bevorzugt 11 - 16, und mit
R¹ = Methyl,

R² = -C_{c}H_{2c}-O-(C₂H₄O-)ₐ(C₃H₆O-)_{b}R⁵

mit
a = 12
b = 0
c = 3
R⁵ = Methyl,
n = 10 - 500,
p = 10 - 50,
ist beispielsweise unter der Handelsbezeichnung Dow Corning 193 (INCI: PEG-12 Dimethicone) erhältlich.

Unter bestimmten Bedingungen kann Dow Corning 193 (PEG-12 Dimethicone) geruchsinstabil sein.

Weitere erfindungsgemäß besonders bevorzugte Zusammensetzungen sind dadurch gekennzeichnet, dass sie PEG/PPG-22/24 Dimethicone und PEG-12 Dimethicone enthalten.

Ein Organosiloxan-Oxyalkylen-Copolymer der allgemeinen Strukturformel (II) mit einem HLB-Wert im Bereich von 8 - 20, bevorzugt 10 - 18, besonders bevorzugt 11 - 16, und mit
R¹ = Methyl,

R² = -C_{c}H_{2c}-O-(C₂H₄O-)ₐ(C₃H₆O-)_{b}R⁵

mit
a=17
b = 18
c= 3
R⁵ = Methyl,
n = 10 - 500,
p = 10 - 50,
ist beispielsweise unter der Handelsbezeichnung Dow Coming Q2-5220 (INCI: PEG/PPG-17/18 Dimethicone) erhältlich.
Weitere erfindungsgemäß besonders bevorzugte Zusammensetzungen sind dadurch gekennzeichnet, dass sie PEG/PPG-22/24 Dimethicone und PEG/PPG-17/18 Dimethicone enthalten.

Ein Organosiloxan-Oxyalkylen-Copolymer der allgemeinen Strukturformel (II) mit einem HLB-Wert im Bereich von 8 - 20, bevorzugt 10 - 18, besonders bevorzugt 11 - 16, und mit
R¹ = Methyl,

R² = -C_{c}H_{2c}-O-(C₂H₄O-)ₐ(C₃H₆O-)_{b}R⁵

mit
a = 20
b = 6
c = 3
R⁵ = Methyl,
n = 10 - 500,
p = 5 - 50,
ist beispielsweise unter der Handelsbezeichnung Abil B 88184 (INCI: PEG/PPG-20/6 Dimethicone) erhältlich. Weitere erfindungsgemäß besonders bevorzugte Zusammensetzungen sind dadurch gekennzeichnet, dass sie PEG/PPG-22/24 Dimethicone und PEG/PPG-20/6 Dimethicone enthalten.

Ein Organosiloxan-Oxyalkylen-Copolymer der allgemeinen Strukturformel (II) mit einem HLB-Wert im Bereich von 8 - 20, bevorzugt 10 - 18, besonders bevorzugt 11 - 16, und mit
R¹ = Methyl,

R² = -C_{c}H_{2c}-O-(C₂H₄O-)ₐ(C₃H₆O-)_{b}R⁵

mit
a= 14
b = 4
c = 3
R⁵ = Methyl,
n= 10 - 500,
p = 5-50,
ist beispielsweise unter der Handelsbezeichnung Abil B 8851 (INCI: PEG/PPG-14/4 Dimethicone) erhältlich.
Weitere erfindungsgemäß besonders bevorzugte Zusammensetzungen sind dadurch gekennzeichnet, dass sie PEG/PPG-22/24 Dimethicone und PEG/PPG-14/4 Dimethicone enthalten.

Ein Organosiloxan-Oxyalkylen-Copolymer der allgemeinen Strukturformel (I) 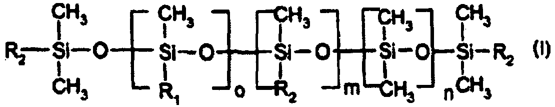 mit einem HLB-Wert im Bereich von 8 - 20, bevorzugt 10 - 18, besonders bevorzugt 11 - 16, und mit
R¹ = Methyl,

R² = -C_{c}H_{2c}-O-(C₂H₄O-)ₐ(C₃H₆O-)_{b}R⁵

mit
a = 20
b = 20
c=3
R⁵ = Methyl,
m = 0,
n = 10 - 500,
ist beispielsweise unter der Handelsbezeichnung Abil B 8832 (INCI: Bis-PEG/PPG-20/20 Dimethicone) erhältlich.
Weitere erfindungsgemäß besonders bevorzugte Zusammensetzungen sind dadurch gekennzeichnet, dass sie PEG/PPG-22/24 Dimethicone und Bis-PEG/PPG-20/20 Dimethicone enthalten.

Ein weiteres bevorzugtes ist Organosiloxan-Oxyalkylen-Copolymer ist Bis-PEG/PPG-16/16 PEG/PPG-16/16 Dimethicone.

Weitere bevorzugte erfindungsgemäße Zusammensetzungen sind dadurch gekennzeichnet, dass das Organosiloxan-Oxyalkylen-Copolymer d) eine Wasserlöslichkeit von mindestens 2 g pro 100 g wässriger Lösung aufweist.
Besonders bevorzugte erfindungsgemäße Zusammensetzungen sind dadurch gekennzeichnet, dass das Organosiloxan-Oxyalkylen-Copolymer d) eine Wasserlöslichkeit von mindestens 5 g pro 100 g wässriger Lösung aufweist.
Weitere bevorzugte erfindungsgemäße Zusammensetzungen sind dadurch gekennzeichnet, dass das Organosiloxan-Oxyalkylen-Copolymer d) zu mindestens 2 Gew.-% mit Wasser mischbar ist. Weitere besonders bevorzugte erfindungsgemäße Zusammensetzungen sind dadurch gekennzeichnet, dass das Organosiloxan-Oxyalkylen-Copolymer d) zu mindestens 5 Gew.-% mit Wasser mischbar ist.
Alle vorstehenden Angaben zur Wasserlöslichkeit bzw. Wassermischbarkeit beziehen sich auf eine Temperatur von 20°C und einen Druck von 1013,25 mbar.

Weitere bevorzugte erfindungsgemäße Zusammensetzungen sind dadurch gekennzeichnet, dass mindestens ein Organosiloxan-Oxyalkylen-Copolymer in einer Gesamtmenge von 0,01 - 5 Gew.-%, bevorzugt 0,1 - 4 Gew.%, besonders bevorzugt in einer Gesamtmenge von 0,5 - 3 Gew.%, außerordentlich bevorzugt in einer Gesamtmenge von 0,7 - 1,5 Gew.-%, enthalten ist, jeweils bezogen auf das Gesamtgewicht der (treibmittelfreien) Zusammensetzung.

Erfindungsgemäß sind die schweißhemmenden Wirkstoffe und gegebenenfalls weitere im Träger unlösliche Wirkstoffe in mindestens einem unter Normalbedingungen flüssigen Öl suspendiert. Zur besseren Anwendbarkeit wird dieser Suspension noch mindestens ein lipophiles Verdickungsmittel als Suspendierhilfe zugesetzt. Weitere bevorzugte erfindungsgemäße Zusammensetzungen sind daher dadurch gekennzeichnet, dass sie mindestens ein lipophiles Verdickungsmittel enthalten.
Bevorzugte erfindungsgemäße Zusammensetzungen sind dadurch gekennzeichnet, dass das mindestens eine lipophile Verdickungsmittel ausgewählt ist aus hydrophobierten Tonmineralien, pyrogenen Kieselsäuren, Bentone-Gelen, Ethylene/Propylene/Styrene Copolymeren, Butylenel Ethylene/Styrene Copolymeren, Dextrinestem, Siliconelastomeren, unter Normalbedingungen festen Wachsen und/oder Glycerintriestern. Hierunter sind hydrophobierte Tonmineralien besonders bevorzugt. Die erfindungsgemäßen Zusammensetzungen enthalten in einer bevorzugten Ausführungsform mindestens ein Suspensions- oder Verdickungsmittel. Besonders geeignete Verdickungsmittel sind hydrophobierte Tonmineralien wie Montmorillonite, Hectorite und Bentonite, insbesondere Disteardimonium Hectorite und Quaternium-18 Hectorite. Die handelsüblichen Verdickungsmittel stellen diese hydrophobierten Tonmineralien als Pulver oder in Form eines vorgefertigten Gels in Cyclomethicone und gewünschtenfalls eines Gelaktivators, wie z. B. Propylencarbonat, bereit. Weitere geeignete Verdickungsmittel sind pyrogene Kieselsäuren, z. B. die Handelsprodukte der Aerosil^{®}-Serie von Degussa.

Bevorzugte hydrophobierte Tonmineralien sind ausgewählt aus hydrophobierten Montmorilloniten, hydrophobierten Hectoriten und hydrophobierten Bentoniten, besonders bevorzugt aus Disteardimonium Hectorite, Stearalkonium Hectorite, Quaternium-18 Hectorite und Quaternium-18 Bentonite. Die handelsüblichen Verdickungsmittel stellen diese hydrophobierten Tonmineralien als Pulver oder in Form eines Gels in einer Ölkomponente, bevorzugt in Cyclomethicone und/oder einer Nichtsilicon-Ölkomponente, wie z. B. Propylencarbonat, bereit. Derartige Pulver oder Gele sind beispielsweise unter der Handelsbezeichnung Bentone^{®} oder Thixogel erhältlich. Erfindungsgemäß bevorzugte Zusammensetzungen sind dadurch gekennzeichnet, dass sie mindestens ein hydrophobiertes Tonmineral in einer Gesamtmenge von 0,5 - 10 Gew.%, bevorzugt 1 - 7 Gew.%, besonders bevorzugt 2 - 6 Gew.%, außerordentlich bevorzugt 3 - 5 Gew.-%, jeweils bezogen auf das Gesamtgewicht der treibmittetfreien erfindungsgemäßen Zusammensetzung, enthalten.
Derartige hydrophobierte Tonmineralien benötigen üblicherweise als Aktivator Ethanol oder Propylencarbonat in einer Menge von 0,3 - 3 Gew.%, bevorzugt 0,5 - 2 Gew.-%, jeweils bezogen auf das Gesamtgewicht der treibmittelfreien erfindungsgemäßen Zusammensetzung.
Weitere erfindungsgemäß bevorzugte lipophile Verdickungsmittel sind ausgewählt aus pyrogenen Kieselsäuren, z. B. den Handelsprodukten der Aerosil^{®}-Serie von Evonik Degussa. Besonders bevorzugt sind hydrophobierte pyrogene Kieselsäuren, besonders bevorzugt Silica Silylate und Silica Dimethyl Silylate.
Erfindungsgemäß bevorzugte Zusammensetzungen sind dadurch gekennzeichnet, dass sie mindestens eine pyrogene Kieselsäure, bevorzugt mindestens eine hydrophobierte pyrogene Kieselsäure, in einer Gesamtmenge von 0,5 -10 Gew.%, bevorzugt 0,8 - 5 Gew.%, besonders bevorzugt 1 - 4 Gew.%, außerordentlich bevorzugt 1,5 - 2 Gew.%, jeweils bezogen auf das Gesamtgewicht der treibmittelfreien erfindungsgemäßen Zusammensetzung, enthalten.
Weitere erfindungsgemäß bevorzugte Zusammensetzungen sind dadurch gekennzeichnet, dass sie mindestens eine hydrophobierte pyrogene Kieselsäure und mindestens eine hydrophile Kieselsäure enthalten.

Weitere erfindungsgemäß bevorzugte lipophile Verdickungsmittel sind ausgewählt aus Ethylene/ Propylene/Styrene Copolymeren und Butylene/Ethylene/Styrene Copolymeren. Besonders bevorzugt werden die Copolymere als vorverdicktes öl-basiertes Gel eingesetzt. Erfindungsgemäß bevorzugte Zusammensetzungen sind dadurch gekennzeichnet, dass sie mindestens ein Ethylene/Propylene/Styrene Copolymer und/oder Butylene/Ethylene/Styrene Copolymer in einer Gesamtmenge von 0,05 - 3 Gew.%, bevorzugt 0,1 - 2 Gew.%, besonders bevorzugt 0,2 - 1,0 Gew.-%, außerordentlich bevorzugt 0,3 - 0,5 Gew.%, jeweils bezogen auf das Gesamtgewicht der treibmittelfreien erfindungsgemäßen Zusammensetzung, enthalten.

Weitere erfindungsgemäß bevorzugte lipophile Verdickungsmittel sind ausgewählt aus Siliconelastomeren. Eine weitere bevorzugte Ausführungsform der Erfindung ist dadurch gekennzeichnet, dass mindestens ein Siliconelastomer, das erhältlich ist durch die Vernetzung eines Organopolysiloxans, das mindestens 2 C₂ - C₁₀ -Alkenylgruppen mit terminaler Doppelbindung in jedem Molekül enthält, mit einem Organopolysiloxan, das mindestens 2 Silicon-gebundene Wasserstoffatome in jedem Molekül aufweist, enthalten ist.
Erfindungsgemäß besonders bevorzugte Organopolysiloxane mit mindestens 2 C₂ - C₁₀ - Alkenyl-Gruppen mit terminaler Doppelbindung im Molekül sind ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.
Erfindungsgemäß besonders bevorzugte vernetzende Organopolysiloxane mit mindestens zwei Silicon-gebundenen Wasserstoffatomen sind ausgewählt aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-siloxan-Copolymeren.

Erfindungsgemäß besonders bevorzugte Siliconelastomere, die als Rohstoff bereits in einem bei Raumtemperatur unter Normalbedingungen flüssigen Silicon vorgequollen vorliegen und ein Silicon-basiertes Gel darstellen, sind kommerziell erhältlich, beispielsweise unter den Handelsnamen SFE 168, ein Cyclomethicone (and) Dimethicone/Vinyl Dimethicone Crosspolymer von GE Silicones, Vinyl Dimethicone Crosspolymere, enthalten in KSG-15 (Cyclomethicone (and) Dimethicone/Vinyl Dimethicone Crosspolymer, Siliconelastomergehalt 4 - 10 Gew.-%), KSG-16 (Dimethicone (and) Dimethicone/Vinyl Dimethicone Crosspolymer, Siliconelastomergehalt 20 - 30 Gew.-%), KSG-17 (Cyclomethicone (and) Dimethicone/Vinyl Dimethicone Crosspolymer), KSG-18 (Phenyl Trimethicone (and) Dimethicone/Phenyl Vinyl Dimethicone Crosspolymer, Siliconelastomergehalt 10 - 20 Gew.-%); and KSG-20, erhältlich von Shin Etsu Silicones of America (Akron, Ohio), und von Grant Industries Inc. (Elmwood Park, NJ) die Produkte aus der Gransil^{®}-Serie. Eine weitere bevorzugte Ausführungsform der Erfindung ist dadurch gekennzeichnet, dass das Siliconelastomer durch die Vernetzung eines Organopolysiloxans, das mindestens 2 C₂ - C₁₀-Alkenylgruppen mit terminaler Doppelbindung in jedem Molekül enthält, mit mindestens einem alpha, omega-Dien erhältlich ist Erfindungsgemäß besonders bevorzugte vernetzende alpha, omega-Diene weisen die Formel CH₂=CH(CH₂)ₓCH=CH₂ mit x = 1 - 20 auf. Besonders bevorzugte alpha, omega-Diene sind ausgewählt aus 1,4-Pentadien, 1,5-Hexadien, 1,6-Heptadien, 1,7-Octadien, 1,8-Nonadien, 1,11-Dodecadien, 1,13-Tetradecadien und 1,19-Eicosadien. Erfindungsgemäß bevorzugte Zusammensetzungen sind dadurch gekennzeichnet, dass sie mindestens ein Siliconelastomer in einer Gesamtmenge von 0,05 - 3 Gew.-%, bevorzugt 0,1 - 2 Gew.%, besonders bevorzugt 0,15 - 0,5 Gew.-%, außerordentlich bevorzugt 0,2 - 0,3 Gew.-%, jeweils bezogen auf das Gesamtgewicht der treibmittelfreien erfindungsgemäßen Zusammensetzung, enthalten.

Weitere bevorzugte erfindungsgemäße Zusammensetzungen sind dadurch gekennzeichnet, dass sie mindestens ein Silicongummi enthalten. Überraschend wurde festgestellt, dass durch den Zusatz eines Silicongummis die schweißhemmende und deodorierende Wirkung der erfindungsgemäßen Zusammensetzungen weiter verlängert werden kann. Ohne an diese Theorie gebunden sein zu wollen, wird vermutet, dass das Silicongummi auf der Haut einen Film bildet, durch den die schweißhemmenden Wirkstoffpartikel besser auf der Haut haften.
Ein erfindungsgemäß besonders bevorzugtes Silicongummi ist ausgewählt aus Siliconpolymeren mit der INCI-Bezeichnung Dimethiconol. Bevorzugt werden diese Dimethiconole in einer niedrigkonzentrierten Lösung in Cyclomethicone oder Dimethicone mit einer kinematischen Viskosität von 0,65 cSt bis maximal 10 cSt eingesetzt Besonders bevorzugte Dimethiconole sind von Dow Corning unter den Handelsnamen Dow Corning 1401, Dow Corning 1403 und Dow Corning 1501 erhältlich; diese Produkte enthalten 10 bis 13 Gew.-% Dimethiconol in Cyclomethicone oder Dimethicone.
Erfindungsgemäß bevorzugte Zusammensetzungen sind dadurch gekennzeichnet, dass sie mindestens ein Silicongummi in einer Gesamtmenge von 0,01 - 0,5 Gew.%, bevorzugt 0,05 - 0,2 Gew.%, besonders bevorzugt 0,1 - 0,15 Gew.-%, jeweils bezogen auf das Gesamtgewicht der treibmittelfreien erfindungsgemäßen Zusammensetzung, enthalten.

Die erfindungsgemäßen Zusammensetzungen enthalten mindestens einen Antitranspirant-Wirkstoff.
Bevorzugte Antitranspirant-Wirkstoffe sind ausgewählt aus den wasserlöslichen adstringierenden anorganischen und organischen Salzen des Aluminiums und Zinks bzw. beliebigen Mischungen dieser Salze.
Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind.
Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.
Die Herstellung bevorzugter Antitranspirant-Wirkstoffe ist beispielsweise in US 3887692, US 3904741, US 4359456, GB 2048229 und GB 1347950 offenbart.
Weiterhin bevorzugt sind Aluminiumsesquichlorhydrat, Aluminiumdichlorhydrat, Aluminiumchlorohydrex-Propylenglycol (PG) oder Aluminiumchlorohydrex-Polyethylenglycol (PEG), Aluminium-Glycol-Komplexe, z. B. Aluminium-Propylenglycol-Komplexe, Aluminiumsesquichlorohydrex-PG oder Aluminiumsesquichlorohydrex-PEG, Aluminium-PG-dichlorohydrex oder Aluminium-PEGdichlorohydrex, Aluminiumhydroxid, weiterhin ausgewählt aus Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat und Zinksulfat.

Erfindungsgemäß besonders bevorzugte Antitranspirant-Wirkstoffe sind ausgewählt aus so genannten "aktivierten" Aluminiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet Aktivierte Aluminiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.
Weitere bevorzugte schweißhemmende Wirkstoffe sind aktivierte Aluminiumsalze, wie sie beispielsweise in US 6245325 oder US 6042816 offenbart sind, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Aluminium - Gewichtsverhältnis von 2:1 - 1:20 und bevorzugt 1:1 bis 1:10 bereitzustellen, sowie ein wasserlösliches Calciumsalz in einer solchen Menge, um ein Ca:Al-Gewichtsverhältnis von 1:1 - 1:28 und bevorzugt 1:2 - 1:25 bereitzustellen.
Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1-16 Gew.-%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Aminosäure, dass das Aminosäure zu (AI+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Glycin, dass das Glycin zu AI - Gewichtsverhältnis 2:1 - 1:20, bevorzugt 1:1 -1:10, beträgt. Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.%, bevorzugt 4 - 13 Gew.-% molekular gebundenes Wasser, weiterhin soviel wasserlösliches Calciumsalz, dass das Ca:AI-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.
Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.
Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.
Für die Stabilisierung der schweißhemmenden Salze bevorzugte Hydroxyalkansäuren sind ausgewählt aus Glycolsäure und Milchsäure.
Weitere bevorzugte schweißhemmende Wirkstoffe sind aktivierte Aluminiumsalze, wie sie beispielsweise in US 6902723 offenbart sind, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu AI - Gewichtsverhältnis von 2:1 - 1:20 und bevorzugt 1:1 bis 1:10 bereitzustellen, sowie ein wasserlösliches Strontiumsalz in einer solchen Menge, um ein Sr:Al-Gewichtsverhältnis von 1:1 -1:28 und bevorzugt 1:2 - 1:25 bereitzustellen.
Besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Aminosäure, dass das Aminosäure zu AI - Gewichtsverhältnis 2:1 - 1:20, bevorzugt 1:1 -1:10, beträgt.
Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.%, bevorzugt 4 - 13 Gew.-% molekular gebundenes Wasser, weiterhin soviel wasserlösliches Strontiumsalz, dass das Sr.Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt.
Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Hydroxyalkansäure, dass das Hydroxyalkansäure zu AI - Gewichtsverhältnis 2:1 -1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von AI:X 0,9:1 bis 2, 1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Weitere bevorzugte schweißhemmende Wirkstoffe sind in US 6663854 und US 20040009133 offenbart.
Die schweißhemmenden Wirkstoffe liegen in ungelöster, suspendierter Form vor.
Sofern die schweißhemmenden Wirkstoffe in einem mit Wasser nicht mischbaren Träger suspendiert vorliegen, ist es aus Gründen der Produktstabilität bevorzugt, dass die Wirkstoffpartikel eine zahlenmittlere Partikelgröße von 0,1 - 200 µm, bevorzugt 1 - 50 µm, besonders bevorzugt 3 - 20 µm und außerordentlich bevorzugt 5 - 10 µm, aufweisen. Bevorzugte Wirkstoffpartikel weisen eine volumenmittlere Partikelgröße von 0,2 - 220 µm, bevorzugt 3 - 60 µm, besonders bevorzugt 4 - 25 µm, weiterhin bevorzugt 5 - 20 µm und außerordentlich bevorzugt 10 - 15,5 µm, auf.

Bevorzugte Aluminiumsalze weisen ein molares Metall-zu-Chlorid-Verhältnis von 1,9 -2,1, bzw. für Sesquichlorohydrate von 1,5:1-1,8:1 auf.

Besonders bevorzugte erfindungsgemäße Zusammensetzungen sind dadurch gekennzeichnet, dass der mindestens eine Antitranspirant-Wirkstoff in einer Menge von 5 - 40 Gew.%, bevorzugt 10 - 35 Gew.%, besonders bevorzugt 11 - 28 Gew.-% und außerordentlich bevorzugt 12 - 20 Gew.-%, enthalten ist, bezogen auf das Gesamtgewicht der kristallwasserfreien Aktivsubstanz (USP) in der treibmittelfreien Gesamtzusammensetzung.

In einer besonders bevorzugten Ausführungsform enthält die Zusammensetzung ein adstringierendes Aluminiumsalz, insbesondere Aluminiumchlorohydrat, besonders bevorzugt Aluminiumchlorohydrat mit einer kristallwasserfreien Aktivsubstanz (USP) von 72 - 88 Gew.-%, bezogen auf den Rohstoff tel quel. Bevorzugte nicht-aktivierte Aluminiumchlorohydrate sind beispielsweise pulverförmig als Micro Dry^{®}, Micro Dry^{®} Ultrafine oder Micro Dry^{®}-323 von Summit/Reheis, als Chlorhydrol^{®} (Pulver) sowie in aktivierter Form als Reach^{®} 101, Reach^{®} 103, Reach^{®} 501 von Reheis/Summit oder AACH-7171 von Summit vertrieben wird. Unter der Bezeichnung Reach^{®} 301 wird ein Aluminiumsesquichlorohydrat von Reheis angeboten, das ebenfalls besonders bevorzugt ist

Die erfindungsgemäßen Zusammensetzungen enthalten mindestens ein Öl als Tragerfluid. Erfindungsgemäß bevorzugte Antitranspirant-Zusammensetzungen enthalten 30 - 95 Gew.-%, bevorzugt 40 - 93 Gew.-%, besonders bevorzugt 50 - 90 Gew.-%, außerordentlich bevorzugt 55 - 85 Gew.%, jeweils bezogen auf die gesamte treibmittelfreie Zusammensetzung, an mindestens einem unter Normalbedingungen flüssigen kosmetischen Öl. Auch eine Gesamtmenge an unter Normalbedingungen flüssigen kosmetischen Ölen von 60, 63, 65, 68, 70, 73, 75, 78 oder 80 Gew.-%, jeweils bezogen auf die gesamte treibmittelfreie Zusammensetzung, kann erfindungsgemäß besonders bevorzugt sein, wobei eine Gesamtmenge von 68 - 73 Gew.-% besonders bevorzugt ist.
Bei den kosmetischen Ölen unterscheidet man flüchtige und nicht-flüchtige Öle. Unter nichtflüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 13 - 12000 Pa (0,1 - 90 mm Hg), besonders bevorzugt 15 - 8000 Pa, außerordentlich bevorzugt 300 - 3000 Pa, aufweisen.

Erfindungsgemäß bevorzugte kosmetische Öle sind ausgewählt aus Siliconölen, zu denen z. B. Dialkyl- und Alkylarylsiloxane, wie beispielsweise Cyclopentasiloxan, Cyclohexasiloxan, Dimethylpolysiloxan, niedermolekulares Phenyl Trimethicone und Methylphenylpolysiloxan, aber auch Hexamethyldisiloxan, Octamethyltrisiloxan und Decamethyltetrasiloxan zählen. Besonders bevorzugt sind flüchtige Siliconöle, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning (Dampfdruck bei 20°C ca. 13 - 15 Pa) enthalten sind. Ebenfalls besonders bevorzugt sind flüchtige lineare Siliconöle mit 2 - 10 Siloxaneinheiten, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/oder L₄, bevorzugt solche Mischungen, wie sie z. B. in den Handelsprodukten DC 2-1184, Dow Corning^{®} 200 (0,65 cSt) und Dow Corning^{®} 200 (1,5 cSt) von Dow Corning enthalten sind. Ein weiteres bevorzugtes flüchtiges Siliconöl ist ein niedermolekulares Phenyl Trimethicone mit einem Dampfdruck bei 20°C von etwa 2000 Pa, wie es beispielsweise von GE Bayer Silicones/Momentive unter der Bezeichnung Bayilone Fluid PD 5 erhältlich ist.
Flüchtige Siliconöle sind hervorragend geeignete Trägeröle für erfindungsgemäß bevorzugte Antitranspirant-Zusammensetzungen, da sie ihnen ein angenehmes Hautgefühl und eine geringe Kleideranschmutzung verleihen. Erfindungsgemäß besonders bevorzugte Antitranspirant-Zusammensetzungen sind daher durch einen Gehalt an mindestens einem flüchtigen Siliconöl von 30 - 95 Gew.-%, bevorzugt 40 - 93 Gew.-%, besonders bevorzugt 50 - 90 Gew.-%, außerordentlich bevorzugt 55 - 85 Gew.-%, jeweils bezogen auf die gesamte treibmittelfreie Zusammensetzung, gekennzeichnet.
Neben oder an Stelle des mindestens einen flüchtigen Siliconöls kann auch mindestens ein flüchtiges Nichtsiliconöl enthalten sein. Bevorzugte flüchtige Nichtsiliconöle sind ausgewählt aus C₈-C₁₆-Isoparaffinen, insbesondere aus Isodecan, Isoundecan, Isododecan, Isotridecan, Isotetradecan, Isopentadecan, und Isohexadecan, sowie Mischungen hiervon. Bevorzugt sind C₁₀-C₁₃-Isoparaffin-Mischungen, insbesondere solche mit einem Dampfdruck bei 20°C von etwa 300 - 400 Pa, bevorzugt 360 Pa. Auch dieses mindestens eine flüchtige Nichtsiliconöl ist bevorzugt in einer Gesamtmenge von 30 - 95 Gew.-%, bevorzugt 40 - 93 Gew.-%, besonders bevorzugt 50 - 90 Gew.-%, außerordentlich bevorzugt 55 - 85 Gew.-%, jeweils bezogen auf die gesamte treibmittelfreie Zusammensetzung, enthalten.
Aufgrund des trockeneren Hautgefühls und der schnelleren Wirkstofffreisetzung sind als Trägeröl flüchtige Siliconöle, Isoparaffine, insbesondere Isodecan, Isoundecan, Isododecan, Isotridecan, Isotetradecan, Isopentadecan, Isohexadecan und Isoeicosan, sowie Mischungen von flüchtigen Siliconölen und Isoparaffinen, insbesondere Isododecan, Isohexadecan oder Isoeicosan, besonders bevorzugt.

Erfindungsgemäß bevorzugte Zusammensetzungen sind dadurch gekennzeichnet, dass das mindestens eine unter Normalbedingungen flüssige Träger-Öl b) mindestens ein Isoparaffinöl, insbesondere Isodecan, Isoundecan, Isododecan, Isotridecan, Isotetradecan, Isopentadecan, Isohexadecan und Isoeicosan, umfasst.

Weitere erfindungsgemäß bevorzugte Zusammensetzungen sind dadurch gekennzeichnet, dass das unter Normalbedingungen flüssige Träger-Ol b) eine Mischung aus b)i) einem flüchtigen Siliconöl, ausgewählt aus Cyclomethicone und linearen Polydimethylsiloxanen mit 2 - 10 Siloxaneinheiten, und b)ii) mindestens einem Isoparaffinöl, Isodecan, Isoundecan, Isododecan, Isotridecan, Isotetradecan, Isopentadecan, Isohexadecan und Isoeicosan, umfasst.

Neben den vorgenannten, üblicherweise als "flüchtigen" Siliconölen bezeichneten Substanzen sowie neben den vorgenannten flüchtigen Nichtsiliconölen können erfindungsgemäß besonders bevorzugte Antitranspirant-Zusammensetzungen weiterhin mindestens ein nichtflüchtiges kosmetisches Öl, ausgewählt aus nichtflüchtigen Siliconölen und nichtflüchtigen Nichtsiliconölen, enthalten. Das mindestens eine nichtflüchtige Öl gleicht den negativen Effekt des flüchtigen Öls auf das Rückstandsverhalten erfindungsgemäß bevorzugter Antitranspirant-Zusammensetzungen aus. Durch die relativ schnelle Verdunstung der flüchtigen Öle können feste, unlösliche Bestandteile, insbesondere die Antitranspirantwirkstoffe, auf der Haut als unschöner Rückstand sichtbar werden. Diese Rückstände können erfolgreich mit einem nichtflüchtigen Öl maskiert werden. Außerdem können mit einem Gemisch aus nichtflüchtigem und flüchtigem Öl Parameter wie Hautgefühl, Sichtbarkeit des Rückstands und Stabilität der Suspension feinreguliert und besser an die Bedürfnisse der Verbraucher angepasst werden.
Selbstverständlich ist es ebenfalls möglich, erfindungsgemäße wasserfreie Antitranspirant-Zusammensetzungen mit einem geringen Anteil an flüchtigen Ölen oder sogar ohne flüchtige Öle zu formulieren. Hierzu sind besonders bevorzugt die Esteröle 2-Ethylhexylpalmitat (z. B. Cegesoft^{®} C 24), Hexyldecyllaurat, 2-Ethylhexylstearat, Isopropylmyristat, Isopropylpalmitat und 2-Ethylhexyllaurat, die Benzoesäureester von linearen oder verzweigten C₈₋₂₂-Alkanolen, insbesondere das Handelsprodukt Finsolv^{®} TN (C₁₂-C₁₅-Alkylbenzoat), C₁₂-C₁₅-Alkyllactat und Di-C₁₂-C₁₃-Alkylmalat geeignet Weiterhin kann es besonders bevorzugt sein, erfindungsgemäße wasserfreie Antitranspirant-Zusammensetzungen ohne Cyclomethicone und ohne flüchtige lineare Siliconöle zu formulieren. Auch hierzu sind besonders bevorzugt die Esteröle 2-Ethylhexylpalmitat (z. B. Cegesoft^{®} C 24), Hexyldecyllaurat, 2-Ethylhexylstearat, Isopropylmyristat, Isopropylpalmitat und 2-Ethylhexyllaurat, die Benzoesäureester von linearen oder verzweigten C₈₋₂₂-Alkanolen, insbesondere das Handelsprodukt Finsolv^{®} TN (C₁₂-C₁₅-Alkylbenzoat), C₁₂-C₁₅-Alkyllactat und Di-C₁₂-C₁₃-Alkylmalat geeignet

Bevorzugte nichtflüchtige Siliconöle sind ausgewählt aus höhermolekularen linearen Dimethylpolysiloxanen, im Handel erhältlich z. B. unter der Bezeichnung Dow Corning^{®} 190, Dow Corning^{®} 200 Fluid mit kinematischen Viskositäten (25°C) im Bereich von 5 - 100 cSt, bevorzugt 6 - 50 cSt oder auch 5 - 10 cSt, und Baysilon^{®} 350 M (mit einer kinematischen Viskosität (25°C) von etwa 350 cSt
Erfindungsgemäß ebenfalls bevorzugte Siliconöle sind ausgewählt aus Siliconen der Formel (Sil-1), wobei x ausgewählt ist aus ganzen Zahlen von 1 - 20.

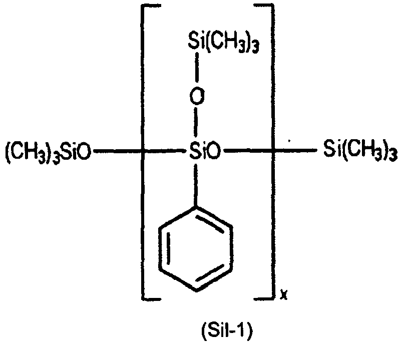

Ein bevorzugtes Siliconöl der Formel (Sil-1) ist unter der INCI-Bezeichnung Phenyl Trimethicone in verschiedenen Qualitäten, Viskositäten und Flüchtigkeiten erhältlich. Ein nicht-flüchtiges Phenyl Trimethicone ist beispielsweise von Dow Corning unter der Bezeichnung Dow Corning 556 erhältlich.

Natürliche und synthetische Kohlenwasserstoffe, wie beispielsweise Paraffinöle, C₁₀-C₃₀-Isoparaffine, insbesondere Isoeicosan, Polyisobutene oder Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (erhältlich z. B. unter dem Handelsnamen Cetiol^{®}S von Cognis) gehören ebenfalls zu den erfindungsgemäß bevorzugten nichtflüchtigen Nichtsiliconölen.
Weitere erfindungsgemäß bevorzugte nichtflüchtige Nichtsiliconöle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD.
Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanoi^{®} G 16, Guerbitol^{®} T 16), Octyldodecanol (Eutanol^{®} G, Guerbitol^{®} 20), 2-Ethylhexylalkohol und die Handelsprodukte Guerbitol^{®} 18, Isofol^{®} 12, Isofol^{®} 16, Isofol^{®} 24, Isofol^{®} 36, Isocarb^{®} 12, Isocarb^{®} 16 oder Isocarb^{®} 24. Weitere bevorzugte nichtflüchtige Nichtsiliconöle sind ausgewählt aus Mischungen aus Guerbetalkoholen und Guerbetalkoholestern, z.B. dem Handelsprodukt Cetiol^{®} PGL (Hexyldecanol und Hexyldecyllaurat).

Weitere erfindungsgemäß bevorzugte nichtflüchtige Nichtsiliconöle sind ausgewählt aus den Triglyceriden von linearen oder verzweigten, gesättigten oder ungesättigten, gegebenenfalls hydroxylierten C₈₋₃₀-Fettsauren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkemöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltrüsostearin und die Handelsprodukte Estol^{®} GTEH 3609 (Uniqema) oder Myritol^{®} GTEH (Cognis) mit verzweigten Fettsäureresten.
Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyll Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Estern der linearen oder verzweigten gesättigten oder nichtflüchtige Nichtsiliconöle ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Isopropylpalmitat, Isopropylstearat, Isopropylmyristat, Hexyldecylstearat (Eutanol^{®} G 16 S), Hexyldecyllaurat, Isononylisononanoat, 2-Ethylhexylpalmitat (Cegesoft^{®} C 24) und 2-Ethylhexylstearat (Cetiol^{®} 868). Ebenfalls bevorzugt sind Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Düsotridecylacetat, n-Hexyllaurat, n-Decyloleat, Oleyloleat, Oleylerucat, Erucyloleat und Erucylerucat.
Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).
Weitere erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether (Ucon Fluid^{®} AP), PPG-9-Butylether (Breox^{®} B25), PPG-10-Butandiol (Macol^{®} 57), PPG-15-Stearylether (Arlamol^{®} E) und Glycereth-7-diisononanoat.
Weitere erfindungsgemäß besonders bevorzugte nichtflüchtige Nichtsiliconöle sind ausgewählt aus den symmetrischen, unsymmetrischen oder cyclischen Estern der Kohlensäure mit Fettalkoholen, z. B. Glycerincarbonat, Dicaprylylcarbonat (Cetiol^{®} CC) oder die Ester gemäß der Lehre der DE 19756454 A1.
Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Es kann erfindungsgemäß bevorzugt sein, Mischungen der vorgenannten Öle einzusetzen. Bevorzugte erfindungsgemäße Zusammensetzungen sind dadurch gekennzeichnet, dass das unter Normalbedingungen flüssige Trägeröl ausgewählt ist flüchtigen Siliconölen, nichtflüchtigen Siliconölen, flüchtigen Kohlenwasserstoffölen, verzweigten gesättigten oder ungesättigten Fettalkoholen mit 6 - 30 Kohlenstoffatomen, Triglyceriden von linearen oder verzweigten, gesättigten oder ungesättigten, gegebenenfalls hydroxylierten C₈₋₃₀-Fettsäuren, Dicarbonsäureestern von linearen oder verzweigten C₂-C₁₀-Alkanolen, Estern von verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können, Anlagerungsprodukten von 1 bis 5 Propylenoxid-Einheiten an ein- oder mehrwertige C₈₋₂₂-Alkanole, Anlagerungsprodukten von mindestens 6 Ethylenoxid und/oder Propylenoxid-Einheiten an ein- oder mehrwertige C₃₋₂₂-Alkanole, C₈-C₂₂-Fettalkoholestern einwertiger oder mehrwertiger C₂-C₇-Hydroxycarbonsäuren, symmetrischen, unsymmetrischen oder cyclischen Estern der Kohlensäure mit Fettalkoholen, den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen, sowie Mischungen der vorgenannten Substanzen.
Es kann erfindungsgemäß außerordentlich bevorzugt sein, Mischungen der vorgenannten Ole einzusetzen, um eine optimale Feinabstimmung der Produkteigenschaften, insbesondere des Rückstandsverhaltens, des Hautgefühls oder der Wirkstofffreisetzung, zu erzielen.

Weitere, erfindungsgemäß besonders bevorzugte Olkomponententypen sind Isoparaffinöle, insbesondere Isododecan, Isohexadecan und Isoeicosan. Isododecan, Isohexadecan und Isoeicosan zählen zu den flüchtigen Ölkomponenten. Da sie nach dem Auftragen auf die Haut relativ schnell verdunsten, reduziert sich die hydrophobe Beladung der schweißhemmenden Wirkstoffpartikel. Derartige flüchtige Ölkomponenten unterstützen so die Freisetzung des schweißhemmenden Wirkstoffs.

Weitere bevorzugte erfindungsgemäße Zusammensetzungen sind dadurch gekennzeichnet, dass mindestens ein Öl in einer Gesamtmenge von 30 - 80 Gew.-%, bevorzugt 40 - 75 Gew.-%, besonders bevorzugt in einer Gesamtmenge von 50 - 73 Gew.%, außerordentlich bevorzugt in einer Gesamtmenge von 56 - 70 Gew.-%, enthalten ist, jeweils bezogen auf das Gesamtgewicht der (treibmittelfreien) Zusammensetzung.

Die Konfektionierung der erfindungsgemäßen Zusammensetzungen, die als Spray appliziert werden, richtet sich vorzugsweise nach den Anforderungen der gewünschten Sprayapplikation. Die erfindungsgemäßen Zusammensetzungen liegen als Suspension vor, das heißt, der schweißhemmende Wirkstoff und gegebenenfalls weitere unlösliche Bestandteile sind in einem flüssigen Träger suspendiert. Ein solches disperses System sollte vor der Anwendung geschüttelt werden. In einer weiteren erfindungsgemäß bevorzugten Ausführungsform sind die erfindungsgemäßen Zusammensetzungen als mit einem Treibmittel versprühbare Suspension konfektioniert.

Bevorzugte erfindungsgemäße Zusammensetzungen können z. B. in Pump- oder Quetschspendern abgepackt sein, insbesondere in Mehrkammer-Pump- oder Quetschspendern. Derartige Spender verwenden Luft, insbesondere die Umgebungsluft, als Treibmittel bzw. fördern die erfindungsgemäße Zusammensetzung durch Pumpen.
In einer weiteren bevorzugten Ausführungsform der Erfindung wird die Zusammensetzung mittels eines komprimierten bzw. verflüssigten Treibmittels appliziert.

Alle Mengenangaben beziehen sich, sofern nicht anders angegeben, auf das Gewicht der treibmittelfreien Zusammensetzung.
Die Verpackung in einem Mehrkammerspender bietet besondere technische Vorteile.
Der Mehrkammerspender kann auch so eingesetzt werden, dass eine Kammer mit der erfindungsgemäßen Zusammensetzung befüllt ist, während eine andere Kammer das komprimierte Treibmittel enthält. Ein derartiger Mehrkammerspender ist beispielsweise eine so genannte Bagin-Can-Verpackung.
Beide Kammern können aber auch so miteinander verbunden, dass die erfindungsgemäße Zusammensetzung in zwei Teilzusammensetzungen aufgetrennt wird, die gleichzeitig aus der Verpackung ausgegeben werden können, beispielsweise aus getrennten Öffnungen oder aus einer einzigen Öffnung.

Weitere bevorzugte erfindungsgemäße Zusammensetzungen sind dadurch gekennzeichnet, dass sie mit mindestens einem Treibmittel in einem geeigneten Druckbehälter verpackt sind. Erfindungsgemäß bevorzugte Treibmittel (Treibgase) sind ausgewählt aus Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, Dichlorfluormethan, Chlordifluormethan, Chlorfluormethan, 1,1,2,2-Tetrachlor-1-fluorethan, 1,1,1,2-Tetrachlor-2-fluorethan, 1,2,2-Trichlor-1,1-difluorethan, 1,1,2-Trichlor-1,2-difluorethan, 1,1,1-Trichlor-2,2-difluorethan, 2,2-Dichlor-1,1.1 -trifluorethan, 1,2-Dichlor-1,1,2-trifluorethan, 2-Chlor-1,1,1,2-tetrafluorethan, 1-Chlor-1,1,2,2-tetrafluorethan, 1,1,2-Trichlor-2-fluorethan, 1,2-Dichlor-1,2-difluorethan, 1,2-Dichlor-1,1-difluorethan, 1-Chlor-1,2,2-trifluorethan, 2-Chlor-1,1,1-trifluorethan, 1-Chlor-1,1,2-trifluorethan, 1,2-Dichlor-1-fluorethan, 1,1-Dichlor-1-fluorethan, 2-Chlor-1,1-difluorethan, 1-Chlor-1,1-difluorethan, 1-Chlor-2-fluorethan, 1-Chlor-1-fluorethan, 2-Chlor-1,1-difluorethen, 1,1,1,3-Tetrafluorethan, Heptafluoro-n-propan, Perfluorethan, Monochlordifluormethan, 1,1-Difluorethan, und zwar sowohl einzeln als auch in Kombination.
Besonders bevorzugt sind Propan, n-Butan, iso-Butan sowie, besonders bevorzugt, Mischungen dieser Treibmittel.
Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann. Die Menge der Treibmittel beträgt bevorzugt 20 - 95 Gew.-%, besonders bevorzugt 30 - 90 Gew.-% und außerordentlich bevorzugt 60 - 86 Gew.%, und weiterhin außerordentlich bevorzugt 75 - 78 Gew.%, jeweils bezogen auf das Gesamtgewicht der Zubereitung, bestehend aus der erfindungsgemäßen Suspension und dem Treibmittel.

Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der erfindungsgemäßen Suspension. Ein erfindungsgemäß bevorzugter Innenschutzlack ist ein Epoxy-Phenollack, wie er u.a. unter der Bezeichnung Hoba 7407 P erhältlich ist. Besonders bevorzugt weisen die verwendeten Ventile einen innenlackierten Ventilteller auf, wobei Lackierung und Ventilmaterial miteinander kompatibel sind. Werden Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein.

### Duftstoffe

Weitere bevorzugte erfindungsgemäße Zusammensetzungen sind dadurch gekennzeichnet, dass sie mindestens einen Duftstoff enthalten. Als Duftstoffe oder Parfümöle können einzelne Riechstoffverbindungen, z.B. die synthetischen Produkte vom Typ der Ester, Ether, Aldehyde, Ketone, Alkohole und Kohlenwasserstoffe verwendet werden. Zu den phenolischen Riechstoffverbindungen zählt z. B. Carvacrol. Riechstoffverbindungen vom Typ der Ester sind z.B. Benzylacetat, Methylanthranilat, ortho-t-Butylcyclohexylacetat, p-tert.-Butylcyclohexylacetat, Diethylphthalat, Nonandiol-1,3-diacetat, iso-Nonylacetat, iso-Nonylformiat, Phenylethylphenylacetat, Phenoxyethylisobutyrat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat, Benzylsalicylat, Ethylsalicylat, iso-Amylsalicylat, Hexylsalicylat und 4-Nonanolid. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. 6-Acetyl-1,1,3,4,4,6-hexamethyltetrahydronaphthalin, para-t-Amylcyclohexanon, 2-n-Heptylcyclopentanon, β-Methylnaphthylketon und die lonone α-Isomethylionon und Methylcedrylketon, zu den Alkoholen Zimtalkohol, Anethol, Citronellol, Dimyrcetol, Eugenol, Geraniol, Linalool, Phenylethylalkohol und Terpineol, zu den Kohlenwasserstoffen gehören 1,3,4,6,7,8-Hexahydro-4,6,6,7,8,8-hexamethylcyclopenta-a-2-benzopyran, Hydroxymethylisopropylcyclopentan, 3-a-Methyldodecahydro-6,6,9a-trimethylnaphtho-2(2,1-b)furan, iso-Butylchinolin sowie die Terpene und Balsame. Bevorzugt werden Mischungen verschiedener Duftstoffe verwendet, die gemeinsam eine ansprechende Duftnote erzeugen.
Geeignete Parfümöle können auch natürliche Riechstoffgemische enthalten, wie sie aus pflanzlichen oder tierischen Quellen zugänglich sind, z.B. Pinien-, Citrus-, Jasmin-, Rosen-, Lilien- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Melissenöl, Minzenöl, Zimtblätteröl, Lindenblotenöl, Wacholderbeerenöl, Vetiveröl, Olibanöl, Galbanumöl, Laudanumöl, Gewürznelkenöl, iso-Eugenol, Thymianöl, Bergamotteöl, Geraniumöl und Rosenöl. Bevorzugte erfindungsgemäße Zusammensetzungen sind dadurch gekennzeichnet, dass mindestens ein Duftstoff in einer Gesamtmenge von 0,1 - 10 Gew.%, bevorzugt 0,2 - 7 Gew.%, besonders bevorzugt 0,4 - 6 Gew.-%, außerordentlich bevorzugt 1 - 5 Gew.-%, weiterhin außerordentlich bevorzugt 2 - 4 Gew.-%, jeweils bezogen auf das Gesamtgewicht der treibmittelfreien Zusammensetzung, enthalten ist

Weitere erfindungsgemäß bevorzugte Zusammensetzungen sind gekennzeichnet durch einen Gehalt an mindestens einem so genannten "hautkühlenden Wirkstoff". Unter hautkühlenden Wirkstoffen im Sinne der vorliegenden Anmeldung werden Wirkstoffe verstanden, die bei Applikation auf die Haut infolge Oberflächenanästhesierung und Reizung der kälteempfindlichen Nerven bei Migräne und dergleichen ein angenehmes Kältegefühl erzeugen, auch wenn die behandelten Hautpartien tatsächlich normale bzw. erhöhte Temperatur zeigen.

Bevorzugte hautkühlende Wirkstoffe sind insbesondere Menthol, Isopulegol sowie Mentholderivate, z. B. Menthyllactat, Menthylpyrrolidoncarbonsäure, Menthylmethylether, Menthoxypropandiol, Menthonglycerinacetal (9-Methyl-6-(1-methylethyl)-1,4-dioxaspiro(4.5)decan-2-methanol), Monomenthylsuccinat und 2-Hydroxymethyl-3,5,5-trimethylcyclohexanol. Als hautkühlende Wirkstoffe besonders bevorzugt sind Menthol, Isopulegol, Menthyllactat, Menthoxypropandiol und Menthylpyrrolidoncarbonsäure.
Bevorzugte erfindungsgemäße Zusammensetzungen enthalten mindestens einen hautkühlenden Wirkstoff in Gesamtmengen von 0,01 - 1 Gew.%, bevorzugt 0,02 - 0,5 Gew.-% und besonders bevorzugt 0,05 - 0,2 Gew.%, jeweils bezogen auf das Gesamtgewicht der (treibmittelfreien) Zusammensetzung.

Bevorzugte erfindungsgemäße Zusammensetzungen sind dadurch gekennzeichnet, dass mindestens ein verkapselter Wirkstoff enthalten ist. Die Wirkstoffe, die vorteilhafterweise verkapselt sein können, sind insbesondere Duftstoffe, Parfümöle und/oder hautkühlende Wirkstoffe, aber auch andere hautpflegende Wirkstoffe, wie Vitamine, Antioxidantien etc.
Als Kapselmaterial bevorzugt sind wasserlösliche Polymere wie Stärke, physikalisch und/oder chemisch modifizierte Stärken, Cellulosederivate, wie z. B. Carboxymethylcellulose, Methylcellulose, Hydroxyethylcellulose oder Hydroxypropylmethylcellulose, Carragheene, Alginate, Maltodextrine, Dextrine, Pflanzengummen, Pektine, Xanthane, Polyvinylacetat und Polyvinylalkohol, Polyvinylpyrrolidin, Polyamide, Polyester und Homo- und Copolymere aus Monomeren, ausgewählt aus Acrylsäure, Methacrylsäure, Maleinsäure, Fumarsäure, Itaconsäure sowie den Estern und den Salzen dieser Säuren, sowie beliebige Mischungen dieser Polymeren.

Bevorzugte Kapselmaterialien sind chemisch modifizierte Stärken, insbesondere Aluminiumstärkeoctenylsuccinat, z. B. das Handelsprodukt Dry Flo Plus von National Starch, oder Natriumstärkeoctenylsuccinat, z. B. das Handelsprodukt Capsul von National Starch, des weiteren Carboxymethylcellulose, Carboxymethylcellulose, Methylcellulose, Hydroxyethylcellulose und Hydroxypropylmethylcellulose, Ethylcellulose, z. B. das Handelsprodukt Tylose H 10 von Clariant, weiterhin Carragheene, Alginate und Maltodextrine, sowie beliebige Mischungen dieser Polymere.

In einer weiteren erfindungsgemäß bevorzugten Ausführungsform enthalten die erfindungsgemäßen Zusammensetzungen 0 bis maximal 5 Gew.-% Ethanol.

Die erfindungsgemäßen Zusammensetzungen sind im Wesentlichen wasserfrei, d. h. sie enthalten 0 bis maximal 3 Gew.-%, bevorzugt 0 bis maximal 2 Gew.-% freies Wasser, bezogen auf die gesamte (treibmittelfreie) Zusammensetzung. Der Gehalt an Kristallwasser, Hydratationswasser oder ähnlich molekular gebundenem Wasser, der in den eingesetzten Bestandteilen, insbesondere in den schweißhemmenden Wirkstoffen enthalten sein kann, stellt im Sinne der vorliegenden Anmeldung kein freies Wasser dar.

Weiterhin können die erfindungsgemäßen Zusammensetzungen zusätzliche Deodorantien enthalten. Als Deodorantien können antimikrobielle, antibakterielle oder keimhemmende Stoffe, Antioxidantien oder Geruchsadsorbentien (z. B. Zinkricinoleat) eingesetzt werden. Geeignete antimikrobielle, antibakterielle oder keimhemmende Stoffe sind insbesondere Organohalogenverbindungen sowie -halogenide, quartäre Ammoniumverbindungen, eine Reihe von Pflanzenextrakten und Zinkverbindungen. Bevorzugt sind halogenierte Phenolderivate wie z. B. Hexachlorophen oder Irgasan DP 300 (Triclosan, 2,4,4'-Trichlor-2'-hydroxydiphenylether), 3,4,4'-Trichlorcarbonilid, Chlorhexidin (1,1'-Hexamethylen-bis-[5-(4-chlorphenyl)]-biguanid), Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar. Auch schwächer wirksame antimikrobielle Stoffe, die aber eine spezifische Wirkung gegen die für die Schweißzersetzung verantwortlichen grampositiven Keime haben, können als Deodorant-Wirkstoffe eingesetzt werden. Auch Benzylalkohol kann als Deodorant-Wirkstoff eingesetzt werden. Weitere antibakteriell wirksame Deodorantien sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide. Bevorzugte Deodorant-Wirkstoffe sind langkettige Diole, z. B. 1,2-Alkan-(C₅-C₁₈)-Diole, Glycerinmono(C₈-C₁₈)-Fettsäureester oder, besonders bevorzugt, Glycerinmono-(C₆-C₁₆)-alkylether, insbesondere 2-Ethylhexylglycerinether, die sehr gut haut- und schleimhautverträglich und gegen Corynebakterien wirksam sind, sowie weiterhin Phenoxyethanol, Phenoxyisopropanol (3-Phenoxy-propan-2-ol), Anisalkohol, 2-Methyl-5-phenyl-pentan-1-ol, 1,1-Dimethyl-3-phenyl-propan-1-ol, Benzylalkohol, 2-Phenyl-ethan-1-ol, 3-Phenylpropan-1-ol, 4-Phenylbutan-1-ol, 5-Phenylpentan-1-ol, 2-Benzylheptan-1-ol, 2,2-Dimethyl-3-phenylpropan-1-ol, 2,2-Dimethyl-3-(3'-methylphenyl)-propan-1-ol, 2-Ethyl-3-phe-nylpropan-1-ol, 2-Ethyl-3-(3'-methylphenyl)-propan-1-ol, 3-(3'-Chlorphenyl)-2-ethylpropan-1-ol, 3-(2'-Chlorphenyl)-2-ethylpropan-1-ol, 3-(4'-Chlorphenyl)-2-ethylpropan-1-ol, 3-(3',4'-Dichlorphenyl)-2-ethylpropan-1-ol, 2-Ethyl-3-(2'-methylphenyl)-propan-1-ol, 2-Ethyl-3-(4'-methylphenyl)-propan-1-ol, 3-(3',4'-Dimethylphenyl)-2-ethylpropan-1-ol, 2-Ethyl-3-(4'-methoxyphenyl)-propan-1-ol, 3-(3',4'-Dimethoxyphenyl)-2-ethylpropan-1-ol, 2-Allyl-3-phenylpropan-1-ol und 2-n-Pentyl-3-phenylpropan-1-ol.

Auch komplexbildende Stoffe können die deodorierende Wirkung unterstützen, indem sie die oxidativ katalytisch wirkenden Schwermetallionen (z. B. Eisen oder Kupfer) stabil komplexieren. Geeignete Komplexbildner sind z. B. die Salze der Ethylendiamintetraessigsäure oder der Nitrilotriessigsäure sowie die Salze der 1-Hydroxyethan-1,1-diphosphonsäure.

Die erfindungsgemäßen Zusammensetzungen können in handelsüblichen Aerosoldosen verpackt sein. Die Dosen können aus Weißblech oder aus Aluminium sein. Weiterhin können die Dosen innen beschichtet sein, um die Gefahr der Korrosion so gering wie möglich zu halten.
Die Dosen sind mit einem geeigneten Sprühkopf ausgestattet. Je nach Sprühkopf sind Ausstoßraten, bezogen auf voll gefüllte Dosen, von 0,1 g/s bis 2,0 g/s möglich.

Unter "Verbesserung der Schweißreduktion" ist erfindungsgemäß sowohl eine Reduzierung der Schweißmenge als auch eine Beschleunigung der Freisetzung des schweißhemmenden Wirkstoffs aus der erfindungsgemäßen Zusammensetzung zu verstehen.

Ein weiterer Gegenstand der vorliegenden Anmeldung ist die Verwendung eines Organosiloxan-Oxyalkylen-Copolymers, ausgewählt aus linearen Polysiloxan-Polyoxyalkylen-Blockcopolymeren, insbesondere aus linearen Polysiloxan-Polyoxyethylen-Polyoxypropylen-Blockcopolymeren, zur Verbesserung der Schweißreduktion einer schweißhemmenden Zusammensetzung gemäß einem der Ansprüche 1 - 8.

Ein weiterer Gegenstand der vorliegenden Anmeldung ist die nicht-therapeutische, kosmetische Verwendung einer schweißhemmenden Zusammensetzung gemäß Anspruch 1, 2, 3, 4, 5, 6, 7 oder 8 zur Reduzierung und/oder Regulierung der Transpiration und/oder des Körpergeruchs.

Bezüglich weiterer bevorzugter Ausführungsformen der erfindungsgemäßen Verwendungen gilt mutatis mutandis das zu den erfindungsgemäßen Zusammensetzungen Gesagte.

Ein weiterer Gegenstand der vorliegenden Anmeldung ist ein nicht-therapeutisches, kosmetisches Verfahren zur Reduzierung und/oder Regulierung der Schweißbildung und/oder des Körpergeruchs, bei dem eine erfindungsgemäße oder erfindungsgemäß bevorzugte Zusammensetzung gemäß einem der Ansprüche 1, 2, 3, 4, 5, 6, 7 oder 8 in einer wirksamen Menge auf die Haut, bevorzugt auf die Haut im Achselbereich, aufgetragen wird.

Bezüglich weiterer bevorzugter Ausführungsformen des erfindungsgemäßen Verfahrens gilt mutatis mutandis das zu den erfindungsgemäßen Zusammensetzungen Gesagte.

Die nachfolgenden Beispiele sollen die Erfindung verdeutlichen, ohne sie hierauf zu beschränken.

Alle Mengenangaben sind in Gew.-%.
Schweißhemmende Suspensions-Zusammensetzungen (Füllgut) zur Konfektionierung als Aerosolspray mit verflüssigtem n-Butan als Treibmittel im Gewichtsverhältnis von Füllgut zu Treibmittel von 10 : 90 bis 30 : 70, bevorzugt von 14 : 86 bis 25 : 75, besonders bevorzugt von 18 : 82 bis 23: 77, weiter bevorzugt 20: 80.

### Die Beispiele Nr. 1 - 8, 12 und 13 sind nicht erfindungsgemäß.

| | Nr. 1 (Vergleich) | Nr. 2 | Nr. 3 | Nr. 4 | Nr. 5 |
|---|---|---|---|---|---|
| Aluminium Chlorohydrate | 33,33 | 33,33 | 33,33 | 33,33 | 33,33 |
| Cyclomethicone, Disteardimonium Hectorite, Propylene Carbonate | 6,67 | 6,67 | 6,67 | 6,67 | 6,67 |
| CYCLOMETHICONE | 55,33 | 53,33 | 53,33 | 53,33 | 53,33 |
| LAURYL GLUCOSIDE | - | - | - | - | 2,00 |
| Bis-PEG/PPG-14/14 Dimethicone | - | 2,00 | - | - | - |
| Bis-PEG/PPG-20/20 Dimethicone | - | - | 2,00 | - | - |
| PEG/PPG-17/18 Dimethicone | - | - | - | 2,00 | - |
| PARFUM (FRAGRANCE) | 4,67 | 4,67 | 4,67 | 4,67 | 4,67 |
| Beurteilung (qualitativ) | 3 | 3,5 | 2,5 | 2 | -* |

| | | | | | |
|---|---|---|---|---|---|
| * LAURYL GLUCOSIDE konnte nicht stabil eingearbeitet und die Zusammensetzung Nr. 5 daher nicht vermessen werden. | | | | | |

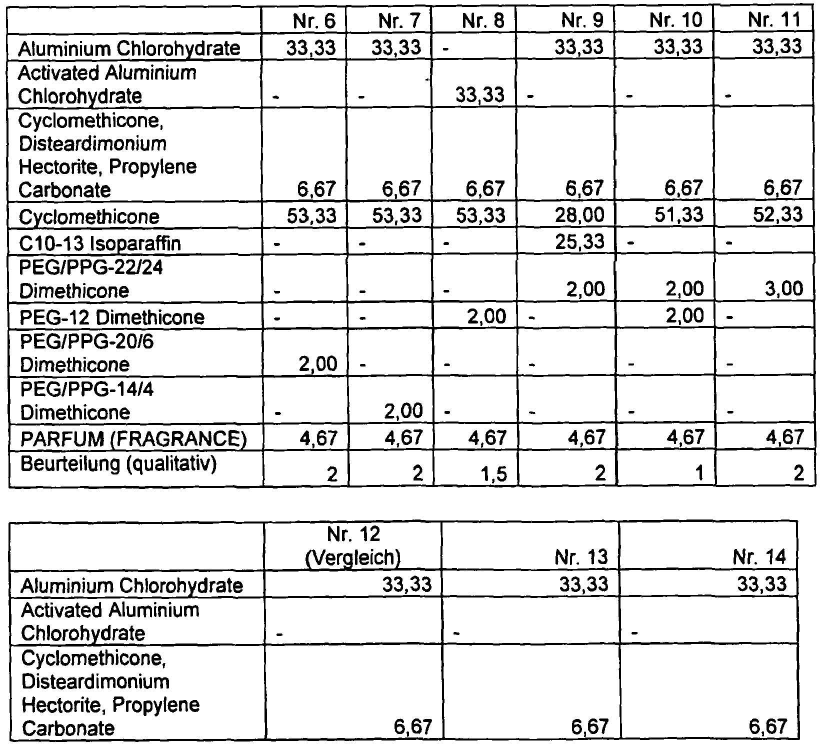

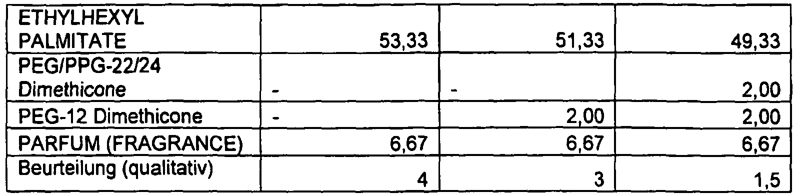

Messung der Freisetzung des schweißhemmenden Wirkstoffs
Zum Nachweis, ob der schweißhemmende Wirkstoff schnell verfügbar ist, wird der zeitliche Verlauf der Leitfähigkeit der Suspensionen aus einem definierten Film in einer bestimmten Menge entionisierten Wassers gemessen.
Der Wert für die am Ende des Versuchs erreichte Leitfähigkeit liegt für die erfindungsgemäßen Zusammensetzungen bei 80 - 160 Mikrosiemens [µS] pro Zentimeter.
Die Zusammensetzungen des Standes der Technik weisen eine finale Leitfähigkeit von maximal 10 - 50 Mikrosiemens pro Zentimeter auf.

Die erfindungsgemäßen und die Vergleichs-Zusammensetzungen Nr. 1 bis Nr. 14 wurden auf die Haut im Achselbereich aufgetragen.

Bei den erfindungsgemäßen Zusammensetzungen wurde eine schneller einsetzende schweißhemmende Wirkung beobachtet.

In den vorstehenden Tabellen ist eine qualitative Beurteilung der getesteten Zusammensetzungen vermerkt, mit einer Notenskala von 1 - 5 mit 1 = sehr gut, 5 = schlecht. In diese Beurteilung geht nicht nur der erreichte Endwert der Leitfähigkeit ein, sondern auch die Steigung der zeitlichen Veränderung der Leitfähigkeit zu Beginn des Versuchs. Eine starke Steigung wird als gleichbedeutend mit einer schnellen Freisetzung des schweißhemmenden Wirkstoffs interpretiert.

**Liste der verwendeten Rohstoffe**

| **INCl-Bezeichnung** | **Rohstoffname** | **Hersteller/Lieferant** |
|---|---|---|
| Aluminium Chlorohydrate | Microdry Ultrafine | Summit Reheis |
| Activated Aluminium Chlorohydrate | Reach 103 | Summit Reheis |
| Cyclomethicone, Disteardimonium Hectorite, Propylene Carbonate | Bentone Gel VS 5 PC V (18 Gew.-% Aktivsubstanz) | Elementis Specialities |
| C10-13 Isoparaffin | Pionier 2094 | |
| ETHYLHEXYL PALMITATE | Cegesoft C 24 | Cognis |
| DI-C12-13 ALKYL MALATE | Cosmacol EMI | Sasol |
| C12-15 ALKYL BENZOATE | Finsolv TN | Innospec (Finetex) |
| PEG-12 Dimethicone | Dow Corning 193 | Dow Corning |
| PEG/PPG-20/6 Dimethicone | Abil B 88184 (100 % Aktivsubstanz) | Evonik |
| PEG/PPG-14/4 Dimethicone | Abil B 8851 | Evonik |
| PEG/PPG-22/24 Dimethicone | Mirasil DMCO (72 Gew.-% PEG/PPG-22/24 Dimethicone Aktivsubstanz), lineares Polysiloxan-Polyoxyalkylen-Blockcopolymer | Rhodia |
| Bis-PEG/PPG-14/14 Dimethicone | Abil EM 97 (83 - 87 Gew.-% Aktivsubstanz) | Evonik |
| Bis-PEG/PPG-20/20 Dimethicone | Abil B 8832 | Evonik |
| PEG/PPG-17/18 Dimethicone | Dow Corning Q2-5220 | Dow Corning |

Weitere erfindungsgemäße Formulierungsbeispiele (Gewichtsverhältnis Füllgut zu Treibmittel wie bei den vorstehenden Beispielen):

| | Nr. 15 | Nr. 16 | Nr. 17 | Nr. 19 | Nr. 20 |
|---|---|---|---|---|---|
| Aluminium Chlorohydrate | - | - | - | - | 33,33 |
| Activated Aluminium Chlorohydrate | 33,33 | 33,33 | 33,33 | 33,33 | - |
| Disteardimonium Hectorite (Bentone Pulver) | 5 | 5 | 5 | 5 | 5 |
| Propylene Carbonate | 1,67 | 1,67 | 1,67 | 1,67 | 1,67 |
| Cyclomethicone | | | | - | - |
| ETHYLHEXYL PALMITATE | 53,33 | 53,93 | - | - | 28,33 |
| Finsolv TN | - | - | 53,33 | 51,33 | 26 |
| PEG/PPG-22/24 Dimethicone | 2 | 0,7 | 1 | 2 | 0,5 |
| PEG-12 Dimethicone | - | - | 1 | - | - |
| PEG/PPG-20/6 Dimethicone | - | - | - | - | - |
| PEG/PPG-14/4 Dimethicone | - | - | - | 2 | - |
| Bis PEG/PPG-20/20 Dimethicone | - | 0,7 | - | - | - |
| PEG/PPG-17/18 Dimethicone | - | - | - | - | 0,5 |
| PARFUM (FRAGRANCE) | 4,67 | 2,67 | 2,67 | 4,67 | 4,67 |
| Encapsulated Fragrance | - | 2 | 2 | - | - |

## Patentansprüche

1. Schweißhemmende Zusammensetzung zur persönlichen Körperpflege, die als treibmittelfrei oder mit einem Treibmittel versprühbare Suspension vorliegt, enthaltend
a) mindestens einen im Träger unlöslichen schweißhemmenden Wirkstoff,
b) mindestens ein unter Normalbedingungen flüssiges Öl als Träger, in dem der schweißhemmende Wirkstoff a) suspendiert ist,
c) 0 - 3 Gew.-%, bevorzugt 0 - 2 Gew.-%, freies Wasser, bezogen auf das Gewicht der treibmittelfreien Zusammensetzung,
d) mindestens ein Organosiloxan-Oxyalkylen-Copolymer, ausgewählt aus linearen Polysiloxan-Polyoxyalkylen-Blockcopolymeren, insbesondere aus linearen Polysiloxan-Polyoxyethylen-Polyoxypropylen-Blockcopolymeren.

2. Zusammensetzung gemäß Anspruch 1, **dadurch gekennzeichnet, dass** das Organosiloxan-Oxyalkylen-Copolymer d) ausgewählt ist aus einem linearen Polysiloxan-Polyoxyethylen-Polyoxypropylen-Blockcopolymer mit der INCl-Bezeichnung PEG/PPG-22/24 Dimethicone.

3. Zusammensetzung gemäß Anspruch 1 oder 2, **dadurch gekennzeichnet, dass** das Organosiloxan-Oxyalkylen-Copolymer d) einen HLB-Wert im Bereich von 8 - 20, bevorzugt 10 - 18, besonders bevorzugt 11 - 16 aufweist.

4. Zusammensetzung gemäß einem der vorstehenden Ansprüche, **dadurch gekennzeichnet, dass** das Organosiloxan-Oxyalkylen-Copolymer d) eine Wasserlöslichkeit von mindestens 5 g pro 100 g wässriger Lösung aufweist.

5. Zusammensetzung gemäß einem der vorstehenden Ansprüche, **dadurch gekennzeichnet, dass** das mindestens eine unter Normalbedingungen flüssige Träger-Öl b) mindestens ein Isoparaffinöl, insbesondere Isodecan, Isoundecan, Isododecan, Isotridecan, Isotetradecan, Isopentadecan, Isohexadecan und Isoeicosan, umfasst.

6. Zusammensetzung gemäß einem der vorstehenden Ansprüche, **dadurch gekennzeichnet, dass** das unter Normalbedingungen flüssige Träger-Öl b) eine Mischung aus b)i) einem flüchtigen Siliconöl, ausgewählt aus Cyclomethicone und linearen Polydimethylsiloxanen mit 2 - 10 Siloxaneinheiten, und b)ii) mindestens einem Isoparaffinöl, Isodecan, Isoundecan, Isododecan, Isotridecan, Isotetradecan, Isopentadecan, Isohexadecan und Isoeicosan, umfasst.

7. Zusammensetzung gemäß einem der vorstehenden Ansprüche, **dadurch gekennzeichnet, dass** das unter Normalbedingungen flüssige Träger-Öl b) eine Mischung aus b)i) mindestens einem flüchtigen Siliconöl und b)ii) mindestens einem Nicht-Siliconöl, ausgewählt aus verzweigten gesättigten oder ungesättigten Fettalkoholen mit 6 - 30 Kohlenstoffatomen, Triglyceriden von linearen oder verzweigten, gesättigten oder ungesättigten, gegebenenfalls hydroxylierten C₈₋₃₀-Fettsäuren, Dicarbonsäureestern von linearen oder verzweigten C₂-C₁₀-Alkanolen, Estern von verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können, Anlagerungsprodukten von 1 bis 5 Propylenoxid-Einheiten an ein- oder mehrwertige C₈₋₂₂-Alkanole, Anlagerungsprodukten von mindestens 6 Ethylenoxid und/oder Propylenoxid-Einheiten an ein- oder mehrwertige C₃₋₂₂-Alkanole, C₈-C₂₂-Fettalkoholestern einwertiger oder mehrwertiger C₂-C₇-Hydroxycarbonsäuren, symmetrischen, unsymmetrischen oder cyclischen Estern der Kohlensäure mit Fettalkoholen, den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen, enthält.

8. Zusammensetzung gemäß einem der Ansprüche 1 - 5, **dadurch gekennzeichnet, dass** kein Cyclomethicone und kein flüchtiges lineares Siliconöl enthalten ist.

9. Zusammensetzung gemäß Anspruch 2, **dadurch gekennzeichnet, dass** weiterhin ein Organosiloxan-Oxyalkylen-Copolymer enthalten ist, ausgewählt aus PEG-12 Dimethicone, PEG/PPG-20/6 Dimethicone, PEG/PPG-14/4 Dimethicone, PEG/PPG-17/18 Dimethicone und Bis-PEG/PPG-20/20 Dimethicone.

10. Nicht-therapeutische, kosmetische Verwendung einer schweißhemmenden Zusammensetzung gemäß einem der Ansprüche 1 - 8 zur Reduzierung und/oder Regulierung der Transpiration und/oder des Körpergeruchs.

11. Nicht-therapeutisches, kosmetisches Verfahren zur Reduzierung und/oder Regulierung der Schweißbildung und/oder des Körpergeruchs, bei dem eine Zusammensetzung gemäß einem der Ansprüche 1 - 8 in einer wirksamen Menge auf die Haut, bevorzugt auf die Haut im Achselbereich, aufgetragen wird.

## Claims

1. An anti-perspirant composition for personal hygiene in the form of a suspension which can be sprayed either using a propellant or not, containing
a) at least one anti-perspirant active ingredient which is insoluble in the carrier,
b) at least one oil that is liquid under normal conditions as the carrier, in which the anti-perspirant active ingredient a) is suspended,
c) 0-3 wt.%, preferably 0-2 wt.% free water, based on the weight of the propellant-free composition,
d) at least one organosiloxane-oxyalkylene copolymer selected from linear polysiloxane-polyalkylene block copolymers, in particular from linear polysiloxane-polyoxyethylene-polyoxypropylene block copolymers.

2. The composition according to claim 1, **characterized in that** the organosiloxane-oxyalkylene copolymer d) is selected from a linear polysiloxane-polyoxyethylene-polyoxypropylene block copolymer having the INCI name PEG/PPG-22/24 dimethicone.

3. The composition according to either claim 1 or claim 2, **characterized in that** the organosiloxane-oxyalkylene copolymer d) has an HLB value in the range of from 8 to 20, preferably 10 to 18, particularly preferably 11 to 16.

4. The composition according to one of the preceding claims, **characterized in that** the organosiloxane-oxyalkylene copolymer d) has a water solubility of at least 5 g per 100 g aqueous solution.

5. The composition according to one of the preceding claims, **characterized in that** the at least one carrier oil b) that is liquid under normal conditions comprises at least one isoparaffinic oil, in particular isodecane, isoundecane, isododecane, isotridecane, isotetradecane, isopentadecane, isohexadecane and isoeicosane.

6. The composition according to one of the preceding claims, **characterized in that** the carrier oil b) that is liquid under normal conditions comprises a mixture of b)i) a volatile silicone oil selected from cyclomethicone and linear polydimethylsiloxanes having 2-10 siloxane units, and b)ii) at least one isoparaffinic oil, isodecane, isoundecane, isododecane, isotridecane, isotetradecane, isopentadecane, isohexadecane and isoeicosane.

7. The composition according to one of the preceding claims, **characterized in that** the carrier oil b) that is liquid under normal conditions contains a mixture of b)i) at least one volatile silicone oil and b)ii) at least one non-silicone oil selected from branched, saturated or unsaturated fatty alcohols having from 6 to 30 carbon atoms, triglycerides of linear or branched, saturated or unsaturated, optionally hydroxylated C₈₋₃₀ fatty acids, dicarboxylic acid esters of linear or branched C₂-C₁₀ alkanols, esters of branched, saturated or unsaturated fatty alcohols having from 2 to 30 carbon atoms having linear or branched, saturated or unsaturated fatty acids having from 2 to 30 carbon atoms which may be hydroxylated, addition products of from 1 to 5 propylene oxide units on monovalent or polyvalent C₈₋₂₂ alkanols, addition products of at least 6 ethylene oxide units and/or propylene oxide units on monovalent or polyvalent C₃₋₂₂ alkanols, C₈₋₂₂ fatty alcohol esters of monovalent or polyvalent C₂-C₇ hydroxycarboxylic acids, symmetrical, asymmetrical or cyclic esters of carboxylic acids having fatty alcohols, the esters of dimers of unsaturated C₁₂-C₂₂ fatty acids (dimer fatty acids) having monovalent linear, branched or cyclic C₂-C₁₈ alkanols or having monovalent linear or branched C₂-C₆ alkanols.

8. The composition according to one of claims 1 to 5, **characterized in that** said composition does not contain cyclomethicone or a volatile linear silicone oil.

9. The composition according to claim 2, **characterized in that** said composition further contains an organosiloxane-oxyalkylene copolymer selected from PEG-12 dimethicone, PEG/PPG-20/6 dimethicone, PEG/PPG-14/4 dimethicone, PEG/PPG-17/18 dimethicone and bis-PEG/PPG-20/20 dimethicone.

10. The non-therapeutic, cosmetic use of an anti-perspirant composition according to one of claims 1 to 8 for reducing and/or controlling perspiration and/or body odor.

11. A non-therapeutic, cosmetic method for reducing and/or controlling the formation of sweat and/or body odor, in which a composition according to one of claims 1 to 8 is applied to the skin, preferably to the skin in the armpit area, in an active amount.

## Revendications

1. Composition anti-transpirante pour les soins corporels personnels, qui se présente sous la forme d'une suspension pulvérisable avec ou sans agent propulseur, contenant
a) au moins une substance active anti-transpirant insoluble dans le support,
b) au moins un huile, liquide dans les conditions normales, qui est utilisée comme support et dans laquelle la substance active anti-transpirante a) est suspendue,
c) de 0 à 3% en poids, de préférence de 0 à 2% en poids, d'eau libre, par rapport au poids de la composition sans agent propulseur,
d) au moins un copolymère organosiloxane-oxyalkylène choisi parmi les copolymères à blocs linéaires polysiloxane-polyoxyalkylène, en particulier parmi les copolymères à blocs linéaires polysiloxane-polyoxyéthylène-polyoxypropylène.

2. Composition selon la revendication 1, **caractérisée en ce que** le copolymère organosiloxane-oxyalkylène d) est choisi parmi un copolymère à blocs linéaire polysiloxane-polyoxyéthylène-polyoxypropylène portant le nom INCl PEG/PPG-22/24 diméthicone.

3. Composition selon la revendication 1 ou 2, **caractérisée en ce que** le copolymère organosiloxane-oxyalkylène d) a une valeur HLB dans la gamme de 8 à 20, de préférence de 10 à 18, de manière particulièrement préférée de 11 à 16.

4. Composition selon l'une des revendications précédentes, **caractérisée en ce que** le copolymère organosiloxane oxyalkylène d) a une solubilité dans l'eau d'au moins 5 g pour 100 g de solution aqueuse.

5. Composition selon l'une des revendications précédentes, **caractérisé en ce qu'**au moins une huile support b), liquide dans les conditions normales, comprend au moins une huile d'isoparaffine, en particulier de l'isododécane, de l'isoundécane, de l'isododécane, de l'isotridécane, de l'isotétradecane, de l'isopentadécane, de l'isohexadécane et de l'isoeicosane.

6. Composition selon l'une des revendications précédentes, **caractérisée en ce que** l'huile support b), liquide dans des conditions normales, comprend un mélange constitué b)i) d'une huile de silicone volatile choisie parmi les cyclométhicone et les polydiméthylsiloxanes linéaires ayant 2 à 10 unités de siloxane, et b)ii) au moins une huile d'isoparaffine, telle que l'isododécane, l'isoundécane, l'isododécane, l'isotridécane, l'isotétradecane, l'isopentadécane, l'isohexadécane et l'isoeicosane.

7. Composition selon l'une des revendications précédentes, **caractérisée en ce que** l'huile support b), liquide dans des conditions normales, comprend un mélange constitué b)i) d'au moins une huile de silicone volatile, et b)ii) d'au moins une huile non siliconée choisie parmi les alcools gras ramifiés, saturés ou insaturés, ayant de 6 à 30 atomes de carbone, des triglycérides d'acides gras en C₈ à C₃₀ linéaires ou ramifiés, saturés ou insaturés, éventuellement hydroxylés, des esters d'acides dicarboxyliques d'alcanols en C₂ à C₁₀ linéaires ou ramifiés, des esters d'alcools gras ramifiés, saturés ou insaturés, ayant 2 à 30 atomes de carbone, ayant 2 à 30 atomes de carbone avec des acides gras linéaires ou ramifiés, saturés ou insaturés, ayant 2 à 30 atomes de carbone, qui peuvent être hydroxylés, des produits d'addition de 1 à 5 unités d'oxyde de propylène à des alcanols en C₈ à C₂₂ monovalents ou polyvalents, des produits d'addition d'au moins 6 unités d'oxyde d'éthylène et/ou d'oxyde de propylène à des alcanols en C₃ à C₂₂ monovalents ou polyvalents, des esters d'alcools gras en C₈ à C₂₂ d'acides hydroxy-carboxyliques en C₂ à C₇ monovalents ou polyvalents, des esters symétriques, asymétriques ou cycliques de l'acide carbonique avec des alcools gras, des esters de dimères d'acides gras en C₁₂ à C₂₂ insaturés (acides gras dimères) avec des alcanols en C₂ à C₁₈ monovalents, linéaires, ramifiés ou cycliques ou avec des alcanols en C₂ à C₆ polyvalents linéaires ou ramifiés.

8. Composition selon l'une quelconque des revendications 1 à 5, **caractérisée en ce qu'**elle ne contient aucune cyclométhicone ni aucune huile de silicone linéaire volatile.

9. Composition selon la revendication 2, **caractérisée en ce qu'**elle contient en outre un copolymère organosiloxane-oxyalkylène choisi parmi PEG-12 Dimethicone, PEG/PPG-20/6 diméthicone, PEG/PPG-14/4 diméthicone, PEG/PPG-17/18 diméthicone et bis-PEG/PPG-20/20 diméthicone.

10. Utilisation cosmétique non-thérapeutique d'une composition anti-transpirante selon l'une des revendications 1 à 8 pour réduire et/ou réguler la transpiration et/ou l'odeur corporelle.

11. Procédé cosmétique non-thérapeutique de réduction et/ou de régulation de la transpiration et/ou de l'odeur corporelle, dans lequel une composition selon l'une des revendications 1 à 8 est appliquée dans une quantité efficace sur la peau, de préférence sur la peau dans la région des aisselles.
